# EUROPEAN PATENT APPLICATION

(11) **EP 3 610 826 A1**
(43) Date of publication of application: **19.02.2020**
(21) Application number: 17914335.9
(22) Date of filing: 05.07.2017
(51) Int. Cl.: A61C 8/00

(54) **FIXATION METHOD FOR DENTAL IMPLANT HOLE PREPARATION MECHANISM AND TOOTH OCCLUSION ADJUSTABLE CONTROL HANDPIECE GUIDE PLATE FOR IMPLEMENTING DENTAL IMPLANT HOLE PREPARATION**

(30) Priority: 22.06.2017 CN 201710482336
(71) Applicant: Shenzhen Ahead Fit Technology Co., Ltd., Shenzhen, Guangdong 518000 (CN)
(72) Inventor: WU, Shengfu, Shenzhen Guangdong 518000 (CN); WU, Meiyan, Shenzhen Guangdong 518000 (CN)
(74) Representative: Sun, Yiming
(86) International application number: PCT/CN2017/091774
(87) International publication number: WO 2018/232783

(57) **Abstract**

The present invention discloses a method for fixing a dental implant hole preparation mechanism and a teeth-biting adjustable-control drill bit guide plate. The method for fixing the dental implant hole preparation mechanism includes: placing the dental implant hole preparation mechanism into an oral cavity first and then getting the dental implant hole preparation mechanism fixed by biting upper teeth and lower teeth jointly. The fixation of a guide plate is implemented by teeth biting of an implant object, so that the fixation difficulty is reduced, and the poor fixation precision and the stability are improved. The teeth-biting adjustable-control drill bit guide plate for implementing dental implant hole preparation includes upper and lower biting plates controlled by upper and lower teeth, and an adjustment mechanism for adjusting the upper biting plate and the lower biting plate multi-directionally. When the teeth-biting adjustable-control drill bit guide plate is used, positions of the upper and lower biting plates are adjusted, so that an implant handpiece drill bit is consistent with a designed implant prepared hole in direction after teeth biting fixation, thus implementing accurate control on an implant handpiece; and as the adjustment to the position of the implant handpiece is achieved by adjusting the positions of the upper and lower biting plates, the teeth-biting adjustable-control drill bit guide plate can be adapted to different dental implant positions only by replacing a tooth die or an alveolar bone die; and therefore, not only is the universality of the guide plate improved, but also the manufacturing time of the implant guide plate can be reduced.

## Description

### FIELD OF TECHNOLOGY

The present invention relates to the technical field of implant guide plates, and in particular to a method for securing a dental implant preparation hole and a bite controlled drill-head guide plate, in preparation of a dental implantation.

### BACKGROUND

A dental implant surgery may provide an important repair option for a tooth loss implant object. During implantation, a doctor needs to use an implant guide plate to determine a position, a depth and a direction of an implant more accurately in the implant surgery.

When the existing implant guide plate is used, it is necessary to keep an oral cavity of the implant object in an open state via other appliances, and the implant guide plate needs to be fixed by virtue of an external force, so the operation of the dental implant surgery is complex, the fixing precision is low and a certain influence is also generated on implant hole preparation.

During tooth implantation, the implant hole preparation is an important step. Since the precision of an implant prepared hole directly affects the implantation effect, the implant prepared hole is highly demanding, i.e., the error between data of an actual implant prepared hole and designed data is required to be small. As a result, an implant handpiece drill bit for making the implant prepared hole is required to be coaxial with a designed implant prepared hole to guarantee the precision of the prepared hole.

Meanwhile, a position in needing of being implanted with a tooth and tooth distribution of each implant object are not completely the same, so an implant prepared hole of each dental implant is different; and thus, the implant handpiece drill bit in each implant hole preparation needs to be adjusted, and the implant guide plate for defining the implant handpiece also needs to be customized according to the tooth distribution of the implant object. The customized implant guide plate is disposable, is abandoned basically after implantation and cannot reused, so that the universality of the implant guide plate is poor, the cost of the dental implant is increased and the resource is wasted. Furthermore, the manufacturing time of each integral dental implant guide plate is also long to affect the delivery cycle of the product.

### TECHNICAL PROBLEM

The technical problem to be solved mainly by the present invention is to provide a method for fixing a dental implant hole preparation mechanism and a teeth-biting adjustable-control drill bit guide plate for implementing dental implant hole preparation. The method for fixing the dental implant hole preparation mechanism can prevent the defect that an implant guide plate needs to be fixed by virtue of an external force in an operation of implant hole preparation, and improves the fixation stability and precision of the dental implant hole preparation mechanism; and meanwhile, with the teeth-biting adjustable-control drill bit guide plate for implementing the dental implant hole preparation, the disposable use of the implant guide plate can be prevented, the universality of most structures is improved, the fixation difficulty of the implant guide plate is reduced, the fixation precision is improved, and the manufacturing time of the implant guide plate is reduced.

### SUMMARY

In order to solve the above technical problems, the present invention provides a method for fixing a dental implant hole preparation mechanism, which includes: placing the dental implant hole preparation mechanism into an oral cavity first and then getting the dental implant hole preparation mechanism fixed by biting upper teeth and lower teeth jointly.

Further, the dental implant hole preparation mechanism is provided with an opening capable of making an implant prepared hole.

The present invention provides a teeth-biting adjustable-control drill bit guide plate for implementing dental implant hole preparation, which includes: an upper biting plate controlled by an upper tooth or an upper alveolar bone and a lower biting plate controlled by a lower tooth or a lower alveolar bone, as well as an adjustment mechanism capable of adjusting the upper biting plate and/or lower biting plate multi-directionally.

Further, the adjustment mechanism includes two upper biting arms connected to the upper biting plate, two lower biting arms connected to the lower biting plate and an adjustment bracket connected to the upper and lower biting arms, as well as two locating plates cooperated with the adjustment bracket to fix the upper and lower biting arms; and one end portion of each of the upper biting arms and/or each of the lower biting arms is provided with a multi-directional moving portion cooperated with a spherical surface of each of the adjustment bracket and the locating plates.

Further, the adjustment mechanism includes two upper biting arms connected to the upper biting plate, two lower biting arms connected to the lower biting plate and an adjustment bracket connected to the upper and lower biting arms, as well as two locating plates cooperated with the adjustment bracket to fix the upper and lower biting arms; a first guide groove is respectively formed on two opposite sides of the adjustment bracket; two first clamping blocks are disposed in each of the first guide grooves; each of the first clamping blocks is contacted and cooperated with a curved surface inside one multi-directional moving portion; a second clamping block is disposed between each of the locating plates and each of the multi-directional moving portions; and the second clamping blocks are contacted and cooperated with spherical surfaces outside the multi-directional moving portions.

Further, each of the multi-directional moving portions is provided with a spherical concave surface and is provided with an outward spherical convex surface; a spherical convex surface and a spherical concave surface of a same multi-directional moving portion are formed into a spherical crown; and spherical crowns on the two lower biting arms are located on two ends of a diameter of a same sphere.

Further, a moving gap is formed between the upper biting arms, the lower biting arms, the locating plates and the adjustment bracket.

Further, areas of the spherical convex surfaces and the spherical concave surfaces of the multi-directional moving portions are respectively greater than contact areas with the locating plates and the adjustment bracket thereof.

Further, the adjustment mechanism includes an upper biting arm connected to the upper biting plate, a lower biting arm connected to the lower biting plate and an adjustment bracket connected to the upper and lower biting arms, as well as locating plates cooperated with the adjustment bracket to fix the upper and lower biting arms; and one end portion of the upper biting arm and/or the lower biting arm is provided with a multi-directional moving portion cooperated with a spherical surface of each of the adjustment bracket and the locating plates.

Further, the adjustment mechanism includes an upper biting arm connected to the upper biting plate, a lower biting arm connected to the lower biting plate and an adjustment bracket connected to the upper and lower biting arms, as well as locating plates cooperated with the adjustment bracket to fix the upper and lower biting arms; end portions of the upper biting arm and/or the lower biting arm are respectively provided with a multi-directional moving portion; the adjustment bracket is further provided with first guide grooves; two first clamping blocks are respectively disposed in the first guide grooves; a second clamping block is disposed between each of the locating plates and each of the multi-directional moving portions; and the first clamping blocks and the second clamping blocks are respectively contacted and cooperated with spherical surfaces of the multi-directional moving portions.

Further, each of the multi-directional moving portions includes a sphere, spherical convex surfaces disposed on two sides symmetrically or spherical concave surfaces disposed on two sides symmetrically.

Further, areas of the spherical convex surfaces or the spherical concave surfaces of the multi-directional moving portions are respectively greater than contact areas with the first clamping blocks and the second clamping blocks thereof.

Further, the adjustment mechanism is provided with a drill bit control mechanism for locating and guiding an implant handpiece handle; the drill bit control mechanism includes a first fixing block and a second fixing block cooperated with the first fixing block; the first fixing block and the second fixing block are respectively provided with a fixing port, and in a cooperated state, the two fixing ports are formed into a fixing hole for clamping and fixing an implant handpiece; and a guide mechanism cooperated with a second guide groove provided by the adjustment bracket is respectively disposed inside the first fixing block and inside the second fixing block, or on two sides of the second fixing block.

Further, a cross section of the first fixing block is of a trapezoidal shape; a fixing groove for accommodating the first fixing block is formed on the second fixing block; and two sides of the fixing groove extend inward respectively to form two block portions for fixing the first fixing block.

Further, each of the guide mechanisms includes two guide bars; and two guide bars on a same inside of the second fixing block are respectively located on two ends of the side of the fixing block.

Further, the adjustment bracket is provided with avoidance grooves cooperated with the guide bars on the first fixing block and the second fixing block.

Further, a limit component for locating the implant handpiece control mechanism is further disposed on the adjustment bracket; the limit component is provided with a slide rail cooperated with two slide grooves of the adjustment bracket; and the limit component is provided with a locating screw.

Further, an implant hole preparation avoidance position is arranged on the upper biting plate and/or lower biting plate located inside a dental implant.

### BENEFICIAL EFFECTS

The dental implant hole preparation mechanism is fixed by biting the upper and lower teeth of the implant object, so that the increase in fixation difficulty and the error in fixation precision due to the fact that the implant guide plate needs to be fixed by virtue of the external force in implant operation are prevented. The teeth-biting adjustable-control drill bit guide plate for implementing the dental implant hole preparation includes the upper and lower biting plates bitten and controlled by the upper and lower teeth or the upper and lower alveolar bones, and the adjustment mechanism capable of adjusting the upper and lower biting plates multi-directionally. When the teeth-biting adjustable-control drill bit guide plate is used, positions of the upper and lower biting plates are adjusted, so that the implant handpiece drill bit is consistent with a designed implant prepared hole in direction after teeth biting fixation. Since the implant guide plate is not fixed by virtue of the external force at the high precision, the difficulty in fixation of the implant guide plate is reduced. As the adjustment to the position of the implant handpiece is achieved by adjusting the positions of the upper and lower biting plates, the accurate control on the implant handpiece can be implemented, the precision of preparing the implant prepared hole is improved, and different dental implant positions can be adapted; for hole preparation of the different dental implant positions, the accurate location can be implemented by only fixing a tooth die or an alveolar bone die consistent with the implant object on the guide plate; and main components are shareable components, so not only is the universality of the guide plate improved, but also the manufacturing time of the implant guide plate can be reduced.

### BRIEF DESCRIPTION OF DRAWINGS

In order to describe the technical solutions in the embodiments of the present invention or in the prior art more clearly, a simple introduction on the accompanying drawings which are needed in the description of the embodiments or the prior art is given below. Apparently, the accompanying drawings in the description below are merely some of the embodiments of the present invention, based on which other drawings may be obtained by those of ordinary skill in the art without any creative effort.
FIG. 1 is a flowchart schematic diagram of a method for fixing a dental implant hole preparation mechanism;
FIG. 2 is an assembly structural schematic diagram of a teeth-biting adjustable-control headpiece bit guide plate in an embodiment;
FIG. 3 is a structural exploded schematic diagram of a teeth-biting adjustable-control headpiece bit guide plate in an embodiment;
FIG. 4 is a structural schematic diagram of a teeth-biting adjustable-control headpiece bit guide plate in use in an embodiment;
FIG. 5 is a structural schematic diagram of a teeth-biting adjustable-control headpiece bit guide plate at another angle in an embodiment;
FIG. 6 is a local structural top view of a teeth-biting adjustable-control headpiece bit guide plate in an embodiment;
FIG. 7 is a structural section view along an A-A direction in FIG. 6;
FIG. 8 is a structural schematic diagram of a teeth-biting adjustable-control headpiece bit guide plate at a still another angle in an embodiment; and
FIG. 9 is a structural section view along a B-B direction in FIG. 8.

The objective implementations, functional characteristics and advantages of the present invention will be further described below in combination with the embodiments and with reference to the accompanying drawings.

### DETAILED DESCRIPTIONS OF EMBODIMENTS

To make the objectives, technical solutions and advantages of the present invention clearer, a clear and complete description of the technical solutions in the present invention will be given below, in combination with the accompanying drawings in the embodiments of the present invention. Apparently, the embodiments described below are a part, but not all, of the embodiments of the present invention. All of the other embodiments, obtained by those of ordinary skill in the art based on the embodiments of the present invention without any inventive efforts, fall into the protection scope of the present invention.

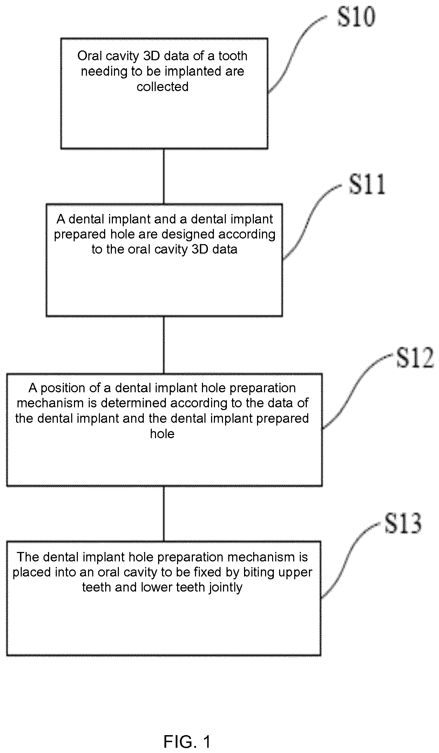

As shown in FIG. 1, the present invention provides an embodiment of a method for fixing a dental implant hole preparation.

The method for fixing the dental implant hole preparation mechanism includes the following steps.

Step S10, oral cavity Three-Dimensional (3D) data of a tooth needing to be implanted are collected, oral cavity 3D data of an implant object are collected, and a dental implant and dental implant prepared hole data model is established.

Step S11, a dental implant and a dental implant prepared hole are designed according to the oral cavity 3D data, and implant data, such as positions, angles and depths, of the dental implant and the dental implant prepared hole for fixing the dental implant, are designed in terms of the dental implant and dental implant prepared hole data model established according to the oral cavity 3D data.

Step S12, a position of a mechanism for making the dental implant prepared hole is determined according to the data of the dental implant and the dental implant prepared hole, and an angle and a movement distance of an implant handpiece drill bit for making the implant prepared hole are determined.

Step S13, the dental implant hole preparation mechanism is placed into an oral cavity to be fixed by biting upper teeth and lower teeth jointly. Specifically, upon the determination of a position of the prepared hole according to the data of the dental implant and the dental implant prepared hole, the dental implant hole preparation mechanism provided with an implant handpiece is placed into the oral cavity. The dental implant hole preparation mechanism is fixed by jointly biting the upper and lower teeth of the implant object, and an opening convenient for the operation of the implant handpiece to make the implant prepared hole is formed.

Specifically, the dental implant hole preparation mechanism is reliably fixed by biting the upper teeth and the lower teeth jointly. After the dental implant hole preparation mechanism is fixed via teeth biting, the opening for making the implant prepared hole can be formed. The opening refers to a space in which the implant handpiece may be used and may be moved in operation of hole preparation at a position needing to be implanted with a tooth. The hole made for fixing a dental implant is referred to as the implant prepared hole.

According to a need, the method for fixing the dental implant hole preparation mechanism further includes a step of making the dental implant prepared hole after the dental implant hole preparation mechanism is fixed. The step of making the dental implant prepared hole includes that through a guide mechanism in sliding fit with the dental implant hole preparation mechanism, the implant handpiece fixed on the guide mechanism is moved to an appropriate position along a designed direction of the dental implant, and the dental implant prepared hole is formed on an alveolar bone.

The data of the dental implant refer to oral cavity 3D data needing to be acquired to implant symmetrically before a tooth is implanted. An adapted appropriate dental implant, as well as an angle, a direction and a depth of the dental implant, is designed according to the oral cavity 3D data.

The dental implant is determined according to the oral cavity 3D data in design, so theoretically speaking, the design data of the dental implant are the most accurate. However, when the dental implant surgery is implemented, since the implant prepared hole is affected by the fixation stability, mechanical precision, operation precision and the like of the dental implant hole preparation mechanism, it is substantially difficult to achieve the complete consistency with the design data of the dental implant.

The implant hole preparation mechanism is reliably fixed by biting the upper and lower teeth jointly, so it does not need to be fixed by virtue of an external force, and an external component or apparatus, and is fixed by the implant object independently, thus reducing the difficulty in fixation of the implant hole preparation mechanism.

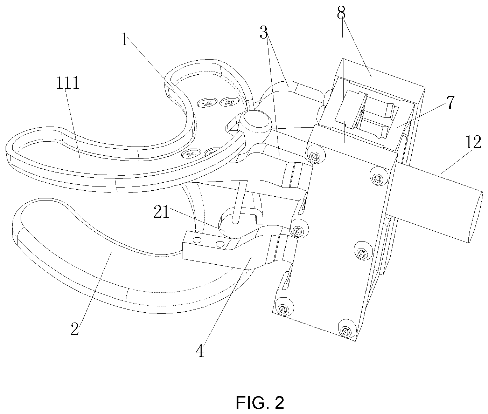

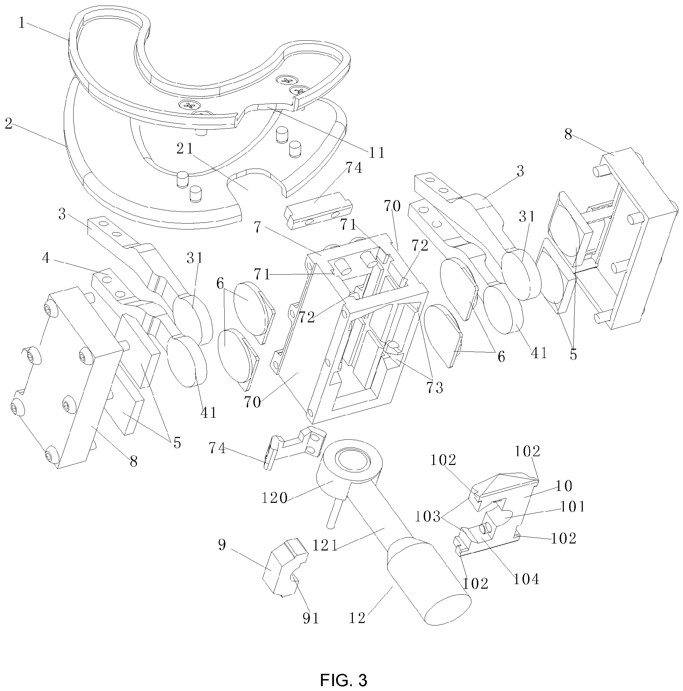

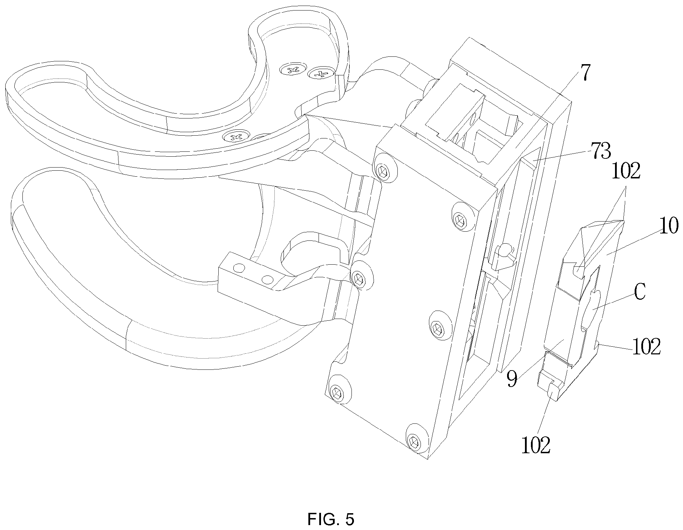

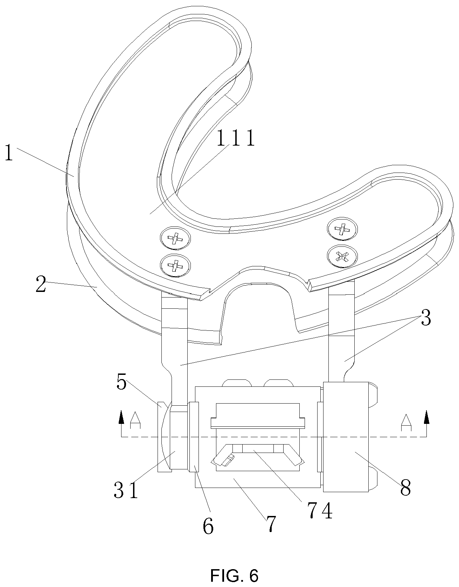

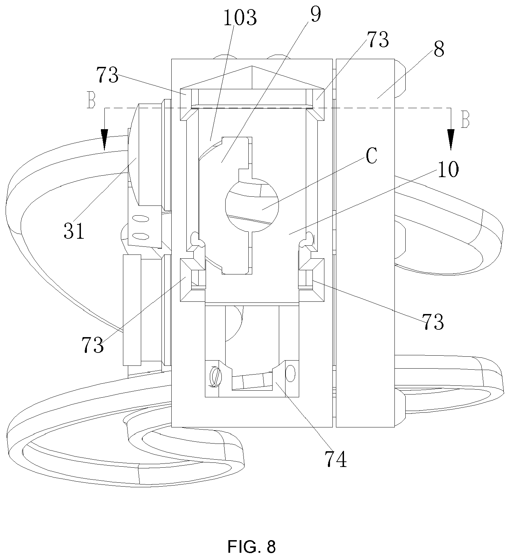

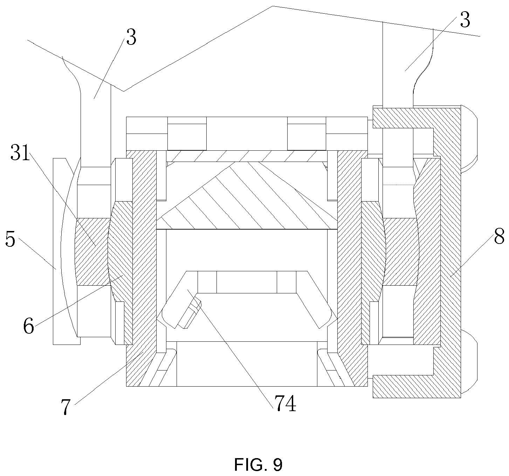

As shown in FIG. 2 to FIG. 9, the present invention provides an embodiment of a teeth-biting adjustable-control drill bit guide plate for implementing dental implant hole preparation.

The teeth-biting adjustable-control drill bit guide plate for implementing the dental implant hole preparation includes: an upper biting plate 1 controlled by an upper tooth or an upper alveolar bone and a lower biting plate 2 controlled by a lower tooth or a lower alveolar bone, as well as an adjustment mechanism capable of adjusting the upper biting plate 1 and/or lower biting plate 2 multi-directionally.

Specifically, the upper biting plate 1 and the lower biting plate 2 are respectively connected to the adjustment mechanism via upper and lower biting arms in a multi-directional and movable manner, i.e., the upper biting plate 1 is fixed with one end of the upper biting arm 3, and the other end of the upper biting arm 3 is connected with the adjustment mechanism in a multi-directional and movable manner. The lower biting plate 2 is fixed with one end of the upper biting arm 4, and the other end of the lower biting arm 4 is connected with the adjustment mechanism in a multi-directional and movable manner. An upper alveolar cavity 111 used for fixing the upper biting plate 1 and cooperated with the upper alveolar bone or the upper tooth is formed on the upper biting plate 1, and a lower alveolar cavity (not shown in the figure) used for fixing the lower biting plate 2 and cooperated with the lower alveolar bone or the lower tooth is formed on the lower biting plate 2. The upper biting plate 1 and the lower biting plate 2 are used for bearing biting forces of the upper and lower teeth or the upper and lower alveolar bones. A tooth die capable of being replaced and cooperated with the tooth or the alveolar bone is arranged on each of the upper biting plate 1 and the lower biting plate 2. The alveolar cavities are the same as the teeth of the implant object. Therefore, the stable and reliable fixation may be formed in occlusion.

The adjustment mechanism takes a fixation action for an implant handpiece handle and takes connection and support actions for the upper and lower biting plates. After the upper biting plate 1 and the lower biting plate 2 are occluded and fixed by adjusting the positions of the upper biting plate 1 and/or the lower biting plate 2, the implant handpiece fixed on the adjustment mechanism is consistent to the designed implant hole preparation data. The adjustment mechanism includes the upper biting arm 3 connected to the upper biting plate 1 and the lower biting arm 4 connected to the lower biting plate 2, as well as an adjustment bracket connected to the upper biting arm 3 and the lower biting arm 4. One end of the upper biting arm 2 is provided with a multi-directional moving portion contacted and cooperated with a curved surface of the adjustment bracket 7, i.e., one end of the upper biting arm 2 is provided with the multi-directional moving portion contacted and cooperated with a spherical surface of the adjustment bracket 7. The upper biting arm 3 is provided with the multi-directional moving portion contacted and cooperated with spherical surfaces of the adjustment bracket 7 and locating plates 8, i.e., the upper biting arm 3 is provided with the multi-directional moving portion 31. The locating plates 8 for tightly clamping and fixing the multi-directional moving portion 31 are disposed on the adjustment bracket 7.

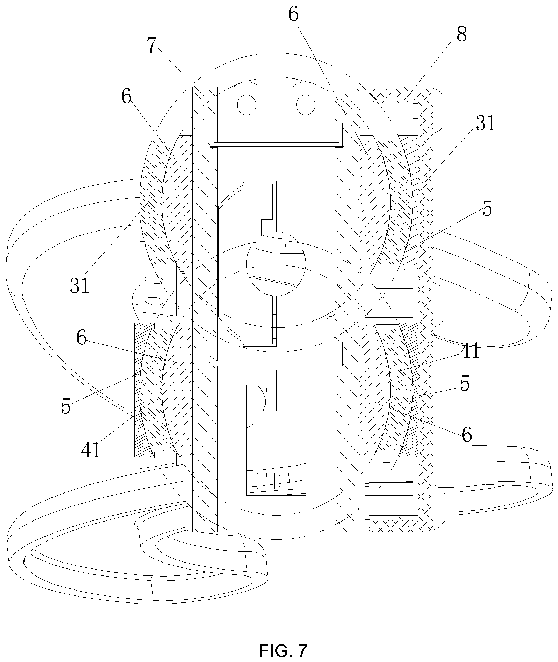

When two upper biting arms 3 are provided, the two upper biting arms 3 are parallel to each other. Each of the multi-directional moving portions is provided with an inward spherical concave surface inside and an outward spherical convex surface outside. A spherical convex surface and a spherical concave surface of a same multi-directional moving portion are formed into a spherical crown. Spherical crowns of the multi-directional moving portions on the two upper biting arms 3 are located on two ends of a diameter of a same sphere. The two spherical crowns are formed into a central symmetry with a spherical center, as shown in FIG. 7. Since the multi-directional moving portions 31 on the two upper biting arms 3 are located on the two ends of the diameter of the same sphere, the two spherical crowns are located on the same sphere, and the two upper biting arms 3 may be moved synchronously in directions such as an up-down rotation direction, a horizontal swing direction or a horizontal rotation direction; and thus, the interference when the two upper biting arms are moved is prevented from generating to affect the movement direction.

In order to guarantee that the multi-directional moving portions each have an enough movement allowance, areas of the spherical convex surfaces and the spherical concave surfaces of the multi-directional moving portions 31 are respectively greater than contact areas with the locating plates 8 and the adjustment bracket 7 thereof. With such an arrangement, the multi-directional moving portions 31 may be guaranteed to contact the locating plates 8 and the adjustment bracket 7 at maximum areas in adjustment. When the multi-directional moving portions 31 are fixed by the locating plates 8, a large action force can be formed, so as to prevent the change in position in teeth biting and thus affect the precision of the implant hole preparation.

In order that the upper biting arms 3 can have a certain swing or rotation allowance in a horizontal direction, a moving gap is formed between the upper biting arms 3, the locating plates 8 and/or the adjustment bracket 7, i.e., the moving gap is respectively formed between the upper biting arms 3 and the locating plates 8, the upper biting arms 3 and the adjustment bracket 7, or the upper biting arms 3, the locating plates 8 and the adjustment bracket 7.

As the adjustment to the position of the upper biting plate 1 is achieved by adjusting the positions of the two upper biting arms 3, after the upper biting plate 1 is adjusted to an appropriate position, the upper biting plate 1 is locked by screws or bolts between the locating plates 8 and the adjustment bracket 7 and thus the positions of the upper biting arms 3 are fixed to implement the location thereof. The symmetrical spherical crowns are adopted by the multi-directional moving portions 31 on the two upper biting arms 3, so not only may the multi-directional adjustment be implemented, but the contact areas with the locating plates and the adjustment bracket are also enabled to be large more easily in location. Therefore, the stable fixation effect is generated more easily in fixation, and the phenomena such as displacement and looseness in teeth biting fixation are prevented from affecting the precision of the implant hole preparation.

Two lower biting arms 4 may also be provided as required. The two lower biting arms are parallel to each other. Each of the lower biting arms is provided with a multi-directional moving portion 41. Herein, the multi-directional moving portions 41 may be the same as the multi-directional moving portions 31 on the upper biting arms 3 in structure. Specifically, each of the multi-directional moving portions 41 is provided with an inward spherical concave surface inside and an outward spherical convex surface outside. A spherical convex surface and a spherical concave surface of a same multi-directional moving portion are formed into a spherical crown. Spherical crowns on the two lower biting arms 4 are located on a same sphere and are respectively located on two ends of a diameter of the same sphere. The spherical crowns on the two lower biting arms 4 are formed into a central symmetry with a spherical center, With the above two lower biting arms 4, it may also be assured that the two upper biting arms can be moved synchronously in up-down rotation, horizontal swing and horizontal rotation, so that the interference when the two upper biting arms are moved is prevented from generating to affect the movement direction.

Likewise, as the adjustment to the position of the lower biting plate 2 is achieved by adjusting the positions of the two lower biting arms 4, after the lower biting plate 2 is adjusted to an appropriate position, the lower biting plate 2 is locked by screws or bolts between the locating plates 8 and the adjustment bracket 7 and thus the positions of the lower biting arms are fixed to implement the location thereof. The symmetrical spherical crowns are adopted by the multi-directional moving portions 41 on the two lower biting arms 4, so the advantages in two aspects are achieved, i.e., not only may the multi-directional adjustment be implemented, but the contact areas with the locating plates and the adjustment bracket are also enabled to be large more easily in location. Therefore, the stable fixation effect is generated more easily in fixation, and the phenomena such as displacement and looseness in teeth biting fixation are prevented from affecting the precision of the implant hole preparation.

In order that the lower biting arms 4 can have a certain swing or rotation allowance in a horizontal direction, a moving gap is formed between the lower biting arms 4, the locating plates 8 and/or the adjustment bracket 7, i.e., the moving gap is respectively formed between the lower biting arms 4 and the locating plates 8, the lower biting arms 4 and the adjustment bracket 7, or the lower biting arms 4, the locating plates 8 and the adjustment bracket 7. Areas of the spherical convex surfaces and the spherical concave surfaces of the multi-directional moving portions 41 are respectively greater than contact areas with the locating plates and the adjustment bracket thereof. With such an arrangement, the multi-directional moving portions 41 may be guaranteed to contact the locating plates 8 and the adjustment bracket 7 at maximum areas all the time in adjustment.

The two lower biting arms and the two upper biting arms may be implemented simultaneously, or either of them may be implemented. Specially, the single or simultaneous implementation is not limited.

In the above each embodiment, the upper biting arms 3 and the lower biting arms 4 cannot be moved horizontally up and down. In order to increase the movement dimension and better adapt to different implant objects, another adjustment mechanism is further provided on the basis of the above embodiment.

The adjustment mechanism includes two upper biting arms 3 connected to the upper biting plate 1, two lower biting arms 4 connected to the lower biting plate 2 and an adjustment bracket 7 connected to the upper and lower biting arms, as well as two locating plates 8 cooperated with the adjustment bracket 7 to fix the upper and lower biting arms; the adjustment bracket is provided with two first guide grooves 70; two first clamping blocks 6 are disposed in each of the first guide grooves 70; each of the first clamping blocks 6 is provided with a spherical convex surface cooperated with a curved surface of one multi-directional moving portion; a second clamping block 5 is disposed between each of the locating plates 8 and each of the multi-directional moving portions; and each of the second clamping blocks 5 is provided with a spherical concave surface cooperated with a curved surface of one multi-directional moving portion.

The first guide grooves 70 are used for guiding and locating the four first clamping blocks 6, so it may be assured that when the two upper biting arms 3 and the two lower biting arms 4 are moved perpendicularly up and down, each of the multi-directional moving portions is located on the same sphere all the time, thus preventing the interference during movement in other directions to fail to implement the movement. Furthermore, it is assured that the upper biting arms 3 and the lower biting arms 4 are not moved back and forth in a horizontal direction, so that a preset offset is prevented from being exceeded between the upper biting plate 1 and the lower biting plate 2 to affect the precision of the teeth biting fixation and the precision of the hole preparation. Other structures and actions not described herein are the same as the foregoing embodiment and will not be repeated.

With the above structure, the two upper biting arms 3 may be guaranteed to rotate synchronously up and down, swing horizontally, rotate horizontally and move horizontally up and down, so that the interference when the two upper biting arms are moved is prevented from generating to affect the movement direction. Likewise, it is also achievable that the two lower biting arms 4 are rotated synchronously up and down, swung horizontally, rotated horizontally and moved horizontally up and down via the embodiment.

In the above embodiment, the fixation on the upper and lower biting plates is implemented by using the two upper biting arms 3 and two lower biting arms 4. As the adjustment to the positions of the two biting plates is implemented by adjusting the positions of the two upper biting arms 3 or the two lower biting arms 4, the precision adjustment of the positions fixed on the adjustment mechanism is implemented to adapt to different implant objects.

On the basis of the two upper biting arms and the two lower biting arms, under a condition in which the upper and lower biting arms can generate enough strength and enough stability is provided between the upper and lower biting arms, the adjustment bracket 7 and the locating plates 8, the present invention further provides a solution in which one upper biting arm and one lower biting arm are adopted to implement the adjustment and location on the positions of the upper and lower biting plates based on the foregoing embodiment.

For example, the adjustment mechanism includes an upper biting arm connected to the upper biting plate, a lower biting arm connected to the lower biting plate and an adjustment bracket connected to the upper and lower biting arms, as well as locating plates cooperated with the adjustment bracket; and end portions of the upper biting arm and the lower biting arm are respectively provided with a multi-directional moving portion contacted and matched with spherical surfaces of the adjustment bracket and the locating plates. The locating plates and the adjustment bracket are used for locating the multi-directional moving portions, which is the same as the foregoing embodiment. When the locating plates and the adjustment bracket are cooperated to generate an enough friction force for the multi-directional moving portions, an enough support force may be provided for the upper biting plate on the upper biting arm and the lower biting plate on the lower biting arm and thus the fixation can also be implemented via teeth biting.

Since the upper biting plate is supported by only one upper biting arm, the upper biting plate is not interfered by other components in multi-directional movement such as up-down rotation, horizontal swing or horizontal rotation of the upper biting arm. In this sense, the multi-directional moving portion may be set into a sphere, two symmetrically-spherical convex surfaces or two symmetrically-spherical concave surfaces.

Likewise, a following structure may also be adopted by the adjustment mechanism as required, i.e., the adjustment mechanism includes an upper biting arm connected to the upper biting plate, a lower biting arm connected to the lower biting plate and an adjustment bracket connected to the upper and lower biting arms, as well as locating plates cooperated with the adjustment bracket; end portions of the upper biting arm and the lower biting arm are respectively provided with a multi-directional moving portion; the adjustment bracket is provided with a guide groove; two first clamping blocks are disposed in the guide groove; each of the first clamping blocks is cooperated with a spherical surface of one multi-directional moving portion; a second clamping block is disposed between each of the locating plates 8 and each of the multi-directional moving portions; and the second clamping blocks are cooperated with spherical surfaces of the multi-directional moving portions. With the above structure, one upper biting arm may be guaranteed to rotate synchronously up and down, swing horizontally, rotate horizontally and move horizontally up and down, so that the interference when the two upper biting arms are moved is prevented from generating to affect the movement direction. Likewise, it is also achievable that one lower biting arm is rotated synchronously up and down, swung horizontally, rotated horizontally and moved horizontally up and down via the embodiment.

The above one lower biting arm and one upper biting arm may be implemented simultaneously, or either of them may be implemented. It may further be appropriate to combine with the foregoing embodiment to implement in a combined manner. Specifically, the implementation using a single or combined manner or other manners is not limited. The action process is the same as the foregoing implementation and will not be repeated.

In the above each implementation, the adjustment mechanism further includes a drill bit control mechanism convenient to implement accurate and stable movement of the implant handpiece, i.e., the drill bit control mechanism implements the location and guidance on the implant handpiece by fixing an implant handpiece handle; and an implant prepared hole may be made in a lateral tooth area in which the opening is small and the operation space is limited.

The drill bit control mechanism includes a first fixing block 9 and a second fixing block 10 cooperated with the first fixing block 9; the first fixing block 9 and the second fixing block 10 are respectively provided with a fixing port, namely, a first fixing port 91 and a second fixing port 101, and in a cooperated state, the first fixing port 91 and the second fixing port 101 are formed into a fixing hole C for clamping and fixing the implant handpiece handle; and a guide mechanism 102 cooperated with a second guide groove 71 provided on the adjustment bracket 7 is respectively disposed outside the first fixing block 9 and outside the second fixing block 10, or on two sides of the second fixing block 10.

Specifically, the drill bit control mechanism fixes the handle 121 of the implant handpiece, which lies in that when the prepared hole is provided in the lateral tooth area, the position of the implant prepared hole is deep, a drill bit 120 of the implant handpiece needs to stretch into the oral cavity, a fixing port in the lateral tooth area is limited to affect the prepared hole, and the drill bit 120 of the implant handpiece can reach to the position of the implant prepared hole only by fixing the handle 121 of the implant handpiece.

Further, a cross section of the first fixing block 9 is of a trapezoidal shape; a fixing groove 104 for accommodating the first fixing block 9 is formed on the second fixing block 10; and two sides of the fixing groove 104 extend inward respectively to form two block portions 103 for fixing the first fixing block 9. In use, the handle 121 of the implant handpiece 12 is placed into the fixing hole C, and then the second fixing block 10 and the first fixing block 9 are locked via screws or bolts, so that the implant handpiece is fixed firmly.

Each of the guide mechanisms 102 includes two guide bars; and two guide bars on a same side of the second fixing block 10 are respectively located on two ends of the side of the fixing block. In this way, the long guidance cooperation can be formed with the second guide groove 71, so that when the guidance cooperation is short, the effect of sliding the precision with a guide bar having a large actual length due to the fact that the clearance precision between the guide bars and the second guide groove 71 is highly demanding is prevented. Furthermore, the large friction in sliding under a condition in which the actual lengths of the guide bars are the same as the length of the first or second fixing block 10 may be reduced. The distance slid by each of the guide mechanisms on the adjustment bracket 7 is limited, so when the guide bars are long, avoidance grooves 73 having widths equal to the lengths of the guide bars need to be reserved; and thus, the guidance cooperation length is small, and a large movement allowance is generated easily to affect the precision when the implant handpiece is moved and then affect the precision of the prepared hole.

The adjustment bracket 7 is provided with the avoidance grooves 73 cooperated with the guide bars on the second fixing block 10 as required. In this way, the drill bit control mechanism fixed with the implant handpiece may be placed to a sliding position more easily.

An inlet end of each of the avoidance grooves 73 is of a horn shape as required, so that the drill bit control mechanism is assembled to a guiding position conveniently.

A limit component 74 for locating the implant handpiece or the drill bit control mechanism is further disposed on the adjustment bracket 7 as required. The limit component 74 is provided with a slide rail cooperated with the two slide grooves 72 of the adjustment bracket 7. The limit component 74 is provided with a locating screw.

With the limit component 74, the depth of the implant handpiece in hole preparation may be set to implement the accurate hole preparation.

In order to better describe the above embodiment, the location of the implant handpiece fixed on the adjustment bracket is implemented by adjusting the positions of the upper and lower biting plates. Specifically, the position of the implant handpiece located on the drill bit control mechanism is fixed, and a linear sliding relationship is present between the drill bit control mechanism and the adjustment bracket 7, i.e., the implant handpiece only can move along the determined direction of the second guide groove 71 provided by the adjustment bracket 7. By adjusting the positions of the upper biting arm 3 and the lower biting arm 4, when a positional relationship between the upper biting plate 1, the lower biting plate 2 and the adjustment bracket 7 changes, the adjustment to an angle and a direction of the drill bit control mechanism to the adjustment bracket 7 is implemented, and thus the adjustment to an angle and a direction of the implant handpiece fixed on the drill bit control mechanism is implemented. When the positional relationship is adjusted to meet the design data of the implant prepared hole, by locking the locating plates 8, the upper and lower biting arms, the adjustment bracket 7 and the implant handpiece are formed into a stable positional relationship; and then, the operation of implant hole preparation is performed, which necessarily meets the design requirement of the implant hole preparation.

According to the need, an upper avoidance position 11 for moving in the implant hole preparation when a lateral tooth area or a rear tooth area is implanted is arranged on the upper biting arm and/or a lower avoidance position 21 for moving in the implant hole preparation when the lateral tooth area or the rear tooth area is implanted is arranged on the lower biting arm, so that the implant handpiece has an enough moving space in the operation.

Hereinafter, based on two upper biting arms, two lower biting arms and a structure in which the upper and lower biting arms may be moved up and down, the work process of a guide plate is described as follows.

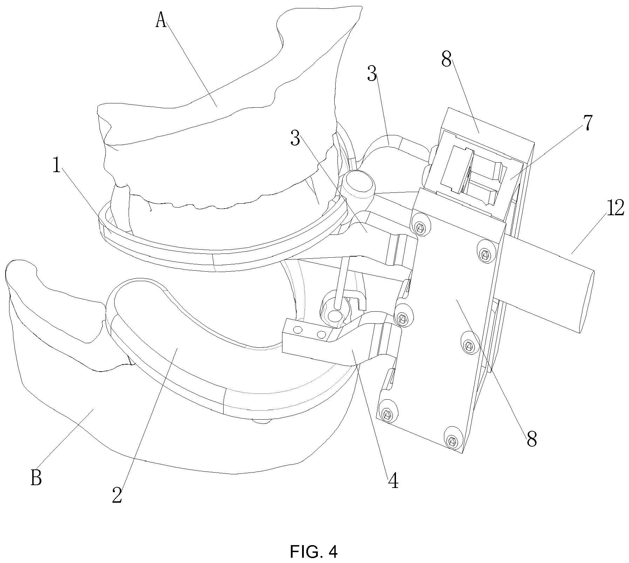

As shown in FIG. 4, the alveolar cavities completely the same as upper and lower alveolar cavities of the implant object are respectively fixed in the upper and lower alveolar cavities, and the implant handpiece and the upper and lower biting plates are adjusted to positions meeting the precision requirements of the implant prepared hole, i.e., by adjusting the positions of the upper biting arms and the lower biting arms, the positional relationship between the upper biting plate, the lower biting plate and the adjustment bracket changes, so that the adjustment to the angle and the direction of the drill bit control mechanism on the adjustment bracket is implemented and thus the adjustment to the angle and the direction of the implant handpiece is implemented. When the positional relationship is adjusted to meet the design data of the dental implant, by locking the locating plates, the positional relationship is stablized; and then, the operation of implant hole preparation is performed, which essentially meets the design requirement of the dental implant.

The guide plate that is well adjusted is placed into the oral cavity of the implant object, and is fixed independently by the implant object via occlusion of an upper tooth A and a lower tooth B. Since a tooth die completely same as teeth of the implant object is respectively arranged on the upper and lower biting plates, the guide plate is not moved or deviated in teeth biting fixation. Then, by moving the implant handpiece fixed on the drill bit control mechanism, the implant handpiece is moved along a direction set by the adjustment bracket, and the implant prepared hole is formed on an alveolar bone. It is appropriate to drill gradually for multiple times with drill bits having different diameters to form the implant prepared hole with the designed size as required. Since the guide plate is adjustable and can be adapted to different implant prepared holes, the applicability is improved. For different implant objects, only a tooth die needs to be replaced; and other structures may be sharable; and therefore, the use cost is reduced and the production time can be reduced.

### INDUSTRIAL APPLICABILITY

The implant hole preparation mechanism can be fixed by the implant object via the occlusion of the upper and lower teeth, so the teeth-biting adjustable-control drill bit guide plate does not need to be fixed by virtue of the external force. As the adjustment to the position of the implant handpiece is implemented by adjusting the positions of the upper and lower biting plates, the teeth-biting adjustable-control drill bit guide plate not only can implement the accurate control on the implant handpiece fixed thereon, but also can be adapted to different dental implant positions. For hole preparation of the different dental implant positions, the accurate location can be implemented by only fixing a tooth die or an alveolar bone die consistent with the implant object on the guide plate; and main components are shareable components, so not only is the universality of the guide plate improved, but the manufacturing time of the implant guide plate also can be reduced.

The foregoing embodiments are merely intended for describing technical solutions of the present invention rather than limiting the present invention. Although the present invention is described in detail with reference to the foregoing embodiments, persons of ordinary skill in the art should understand that they may still make modifications to the technical solutions described in the foregoing embodiments, or make equivalent replacements to some or all technical features thereof; however, these modifications or replacements do not make the essence of corresponding technical solutions depart from the scope of the technical solutions in the embodiments of the present invention.

## Claims

1. A method for fixing a dental implant hole preparation mechanism, comprising: placing the dental implant hole preparation mechanism into an oral cavity first and then getting the dental implant hole preparation mechanism fixed by biting upper teeth and lower teeth jointly.

2. The method for fixing the dental implant hole preparation mechanism according to claim 1, wherein the dental implant hole preparation mechanism is provided with an opening capable of making an implant prepared hole.

3. A teeth-biting adjustable-control drill bit guide plate for implementing dental implant hole preparation, wherein the teeth-biting adjustable-control drill bit guide plate comprises an upper biting plate controlled by an upper tooth or an upper alveolar bone and a lower biting plate controlled by a lower tooth or a lower alveolar bone, as well as an adjustment mechanism capable of adjusting the upper biting plate and/or the lower biting plate multi-directionally.

4. The teeth-biting adjustable-control drill bit guide plate for implementing the dental implant hole preparation according to claim 3, wherein the adjustment mechanism comprises two upper biting arms connected to the upper biting plate, two lower biting arms connected to the lower biting plate and an adjustment bracket connected to the upper and lower biting arms, as well as two locating plates cooperated with the adjustment bracket to fix the upper and lower biting arms; and one end portion of each of the upper biting arms and/or each of the lower biting arms is provided with a multi-directional moving portion cooperated with a spherical surface of each of the adjustment bracket and the locating plates.

5. The teeth-biting adjustable-control drill bit guide plate for implementing the dental implant hole preparation according to claim 3, wherein the adjustment mechanism comprises two upper biting arms connected to the upper biting plate, two lower biting arms connected to the lower biting plate and an adjustment bracket connected to the upper and lower biting arms, as well as two locating plates cooperated with the adjustment bracket to fix the upper and lower biting arms; a first guide groove is respectively formed on two opposite sides of the adjustment bracket; two first clamping blocks are disposed in each of the first guide grooves; each of the first clamping blocks is contacted and cooperated with a curved surface inside one multi-directional moving portion; a second clamping block is disposed between each of the locating plates and each of the multi-directional moving portions; and the second clamping blocks are contacted and cooperated with spherical surfaces outside the multi-directional moving portions.

6. The teeth-biting adjustable-control drill bit guide plate for implementing the dental implant hole preparation according to claim 4 or 5, wherein each of the multi-directional moving portions is provided with a spherical concave surface and is provided with an outward spherical convex surface; a spherical convex surface and a spherical concave surface of a same multi-directional moving portion are formed into a spherical crown; and spherical crowns on the two lower biting arms are located on two ends of a diameter of a same sphere.

7. The teeth-biting adjustable-control drill bit guide plate for implementing the dental implant hole preparation according to claim 6, wherein a moving gap is formed between the upper biting arms, the lower biting arms, the locating plates and the adjustment bracket.

8. The teeth-biting adjustable-control drill bit guide plate for implementing the dental implant hole preparation according to claim 6, wherein areas of the spherical convex surfaces and the spherical concave surfaces of the multi-directional moving portions are respectively greater than contact areas with the locating plates and the adjustment bracket thereof.

9. The teeth-biting adjustable-control drill bit guide plate for implementing the dental implant hole preparation according to claim 3, wherein the adjustment mechanism comprises an upper biting arm connected to the upper biting plate, a lower biting arm connected to the lower biting plate and an adjustment bracket connected to the upper and lower biting arms, as well as locating plates cooperated with the adjustment bracket to fix the upper and lower biting arms; and one end portion of the upper biting arm and/or the lower biting arm is provided with a multi-directional moving portion cooperated with a spherical surface of each of the adjustment bracket and the locating plates.

10. The teeth-biting adjustable-control drill bit guide plate for implementing the dental implant hole preparation according to claim 3, wherein the adjustment mechanism comprises an upper biting arm connected to the upper biting plate, a lower biting arm connected to the lower biting plate and an adjustment bracket connected to the upper and lower biting arms, as well as locating plates cooperated with the adjustment bracket to fix the upper and lower biting arms; end portions of the upper biting arm and/or the lower biting arm are respectively provided with a multi-directional moving portion; the adjustment bracket is further provided with first guide grooves; two first clamping blocks are respectively disposed in the first guide grooves; a second clamping block is disposed between each of the locating plates and each of the multi-directional moving portions; and the first clamping blocks and the second clamping blocks are respectively contacted and cooperated with spherical surfaces of the multi-directional moving portions.

11. The teeth-biting adjustable-control drill bit guide plate for implementing the dental implant hole preparation according to claim 9 or 10, wherein each of the multi-directional moving portions comprises a sphere, spherical convex surfaces disposed on two sides symmetrically or spherical concave surfaces disposed on two sides symmetrically.

12. The teeth-biting adjustable-control drill bit guide plate for implementing the dental implant hole preparation according to claim 11, wherein areas of the spherical convex surfaces or the spherical concave surfaces of the multi-directional moving portions are respectively greater than contact areas with the first clamping blocks and the second clamping blocks thereof.

13. The teeth-biting adjustable-control drill bit guide plate for implementing the dental implant hole preparation according to claim 4, 5, 9, or 10, wherein the adjustment mechanism is provided with a drill bit control mechanism for locating and guiding an implant handpiece handle; the drill bit control mechanism comprises a first fixing block and a second fixing block cooperated with the first fixing block; the first fixing block and the second fixing block are respectively provided with a fixing port, and in a cooperated state, the two fixing ports are formed into a fixing hole for clamping and fixing an implant handpiece; and a guide mechanism cooperated with a second guide groove provided by the adjustment bracket is respectively disposed inside the first fixing block and inside the second fixing block, or on two sides of the second fixing block.

14. The teeth-biting adjustable-control drill bit guide plate for implementing the dental implant hole preparation according to claim 13, wherein a cross section of the first fixing block is of a trapezoidal shape; a fixing groove for accommodating the first fixing block is formed on the second fixing block; and two sides of the fixing groove extend inward respectively to form two block portions for fixing the first fixing block.

15. The teeth-biting adjustable-control drill bit guide plate for implementing the dental implant hole preparation according to claim 13, wherein each of the guide mechanisms comprises two guide bars; and two guide bars on a same inside of the second fixing block are respectively located on two ends of the side of the fixing block.

16. The teeth-biting adjustable-control drill bit guide plate for implementing the dental implant hole preparation according to claim 15, wherein the adjustment bracket is provided with avoidance grooves cooperated with the guide bars on the first fixing block and the second fixing block.

17. The teeth-biting adjustable-control drill bit guide plate for implementing the dental implant hole preparation according to claim 13, wherein a limit component for locating the implant handpiece control mechanism is further disposed on the adjustment bracket; the limit component is provided with a slide rail cooperated with two slide grooves of the adjustment bracket; and the limit component is provided with a locating screw.

18. The teeth-biting adjustable-control drill bit guide plate for implementing the dental implant hole preparation according to claim 3, wherein an implant hole preparation avoidance position is arranged on the upper biting plate and/or the lower biting plate located inside a dental implant.
